(19) Europäisches Patentamt
European Patent Office
Office européen des brevets

(11) **EP 2 407 186 A1**

(12) **EUROPÄISCHE PATENTANMELDUNG**

(43) Veröffentlichungstag:
**18.01.2012 Patentblatt 2012/03**

(51) Int Cl.:
***A61M 1/10*** *(2006.01)*     ***A61M 1/12*** *(2006.01)*

(21) Anmeldenummer: **10075303.7**

(22) Anmeldetag: **15.07.2010**

(84) Benannte Vertragsstaaten:
**AL AT BE BG CH CY CZ DE DK EE ES FI FR GB
GR HR HU IE IS IT LI LT LU LV MC MK MT NL NO
PL PT RO SE SI SK SM TR**
Benannte Erstreckungsstaaten:
**BA ME RS**

(71) Anmelder: **ECP Entwicklungsgesellschaft mbH
12247 Berlin (DE)**

(72) Erfinder:
• **Schumacher, Jörg
14513 Teltow (DE)**
• **Scheckel, Mario
13187 Berlin (DE)**

(74) Vertreter: **Golkowsky, Stefan
Pfenning, Meinig & Partner GbR
Patent- und Rechtsanwälte
Joachimstaler Strasse 10-12
10719 Berlin (DE)**

(54) **Rotor für eine Pumpe, hergestellt mit einem ersten, elastischen Werkstoff**

(57)     Die Erfindung bezieht sich auf einen Rotor (13) für eine Pumpe, mit wenigstens einem Schaufelblatt (19, 20, 22, 29, 30, 31, 32), wobei der Rotor zur Förderung eines Fluids in axialer oder radialer Richtung um eine Rotationsachse (21) rotierend antreibbar ist und wobei der Rotor in radialer Richtung zwischen einem ersten, radial komprimierten Zustand und einem zweiten, radial expandierten Zustand reversibel elastisch verformbar ist.

Fig. 2

EP 2 407 186 A1

**Beschreibung**

Die Erfindung liegt auf dem Gebiet der Mechanik und der Feinwerktechnik und bezieht sich auf Rotoren für Fluidpumpen.

Derartige Pumpen sind vielfältig auf verschiedenen Gebieten einsetzbar. Speziell für den Einsatz in schwierig zugänglichen Umfeldern kann es sinnvoll sein, komprimierbare und expandierbare Rotoren einzusetzen, um diese in komprimiertem Zustand an einen Einsatzort zu bringen und dort zu expandieren, um sie in Betrieb zu nehmen. Spezielle Anwendungen liegen beispielsweise bei Pumpen für verwinkelte Leitungssysteme.

Eine weitere spezielle Anwendung solcher Pumpen findet auf dem medizinischen Gebiet statt. Dort werden Pumpen insbesondere im invasiven Bereich eingesetzt, d. h. eingebracht in den Körper eines Patienten. Es ist bekannt, Pumpen in verschiedenen Formen, beispielsweise als Katheterpumpen, durch körpereigene Gefäße, beispielsweise Blutgefäße, zu befördern und sie dort oder beispielsweise auch in einer Herzkammer zu betreiben. Für solche speziellen Anwendungen muss eine entsprechende Pumpe besonders klein aufgebaut sein, was den radialen Durchmesser angeht.

Um solche Pumpen einfach komprimierbar und expandierbar zu gestalten, sind verschiedene Maßnahmen möglich. So sind beispielsweise Mechanismen bekannt geworden, um Schaufelblätter an entsprechenden Pumpenrotoren gelenkig und radial aus- und anklappbar zu gestalten. Es sind auch wenigstens teilweise aus sogenannten Gedächtnismaterialien bestehende Rotoren bekannt geworden, die ihre Gestalt in Abhängigkeit von der Umgebungstemperatur ändern und auf diese Weise expandierbar oder komprimierbar sind.

Aus der US 2008/0114339 A1 ist beispielsweise ein komprimierbarer und expandierbarer Rotor (Impeller) mit einer Nabe bekannt, die verschiedene Schaufelblätter trägt. Nabe und Schaufelblätter können aus demselben Werkstoff bestehen, der elastisch ausgebildet ist, so dass die Schaufelblätter zwischen einer komprimierten, an die Nabe angelegten Stellung und einer expandierten, radial aufgestellten Stellung bewegbar sind. Dafür ist eine hohe Elastizität des Materials notwendig. Es werden dort beispielsweise besonders nachgiebige Polyurethane als Material vorgeschlagen. Grundsätzlich sollen auch elastische Polymere einsetzbar sein.

Grundsätzlich soll der Rotor gemäß dem genannten Dokument zwischen der komprimierten und der expandierten Gestalt einerseits in einem linearen Bereich der Spannungs-Dehnungs-Kurve und zudem in einem nichtlinearen Bereich der Spannungs-Dehnungs-Kurve verformt werden, um den großen notwendigen Verformungshub vollziehen zu können.

Vor dem Hintergrund des Standes der Technik liegt der vorliegenden Erfindung die Aufgabe zugrunde, einen Rotor der eingangs genannten Art zu schaffen, der in hohem Maße komprimierbar und expandierbar ist und bei dem auch bei wiederholter Kompression und Expansion keine bleibenden Materialveränderungen entstehen.

Die Aufgabe wird gemäß der Erfindung mit den Merkmalen des Patentanspruchs 1 gelöst.

Hierzu ist bei einem Rotor, der zur Förderung eines Fluids um eine Rotationsachse rotierend antreibbar ist und der das Fluid in axialer oder radialer Richtung antreibt, vorgesehen, dass der Rotor in radialer Richtung zwischen einem ersten radial komprimierten Zustand und einem zweiten, radial expandierten Zustand reversibel elastisch verformbar ist, möglichst mit minimierter Hysterese oder optimal ganz ohne eine Hysterese.

Hierzu besteht der Rotor in einer Ausführungsform wenigstens teilweise aus einem ersten, elastischen Werkstoff in Form eines Schaumstoffpolyurethans, eines massiven Polyurethans, eines thermoplastischen Elastomers, eines Kautschuks oder eines superelastischen Werkstoffs, insbesondere superelastischen Polymers.

Ein kraftfreier Zustand des Rotors, d. h. die Stellung, in die er ohne das Einwirken äußerer Kräfte zurückfällt, ist vorteilhaft der zweite Zustand, und er ist weitgehend unabhängig davon, ob dieser kraftfreie Zustand vom komprimierten oder vom expandierten Zustand aus erreicht wird.

Vorteilhaft umfasst der erste Werkstoff ein Polyurethan auf der Basis eines Diisocyanats. Das Polyurethan kann vorteilhaft mit einem Polyetherpolyol hergestellt sein, insbesondere einem Polyetherpolyol mit 2 bis 8 OH-Gruppen pro Molekül. Derartige Polyetherpolyole sind aus zwei- und mehrwertigen Alkoholen und Propylen- und/oder Ethylenoxid herstellbar.

Es kann grundsätzlich ein organisch gefülltes Polyol, insbesondere ein Pfropf-, SAN- oder Polymerpolyol oder ein PHD-Polyol verwendet werden. Diese Polyole können zur Steigerung der Elastizität des ersten Werkstoffs eingesetzt werden.

Besonders vorteilhaft kann der erste Werkstoff ein thermoplastisches Elastomer (TPE) umfassen, insbesondere ein Polyamid-TPE (TPA), ein Copolyester-TPE (TPC), ein Styrol-TPE (TPS), ein Urethan-TPE (TPU), ein TPE mit vernetztem Kautschuk (TPV) oder ein Acrylnitril/Butadien-Kautschuk + Polyvinylchlorid (TPZ).

Speziell kommt als Polyamid-TPE (TPA) ein thermoplastisches Polyamidelastomer in Frage, bei dem die Weichsegmente mit Ether- und Esterbindungen ausgeführt sind, oder ein thermoplastisches Polyamidelastomer, bei dem die Weichsegmente als Polyester (TPA-ES) oder als Polyether (TPA-ET) ausgeführt sind. Bei den Copolyesterelastomeren (TPC) können die Weichsegmente ebenfalls aus Ether- und Esterbindungen (TPC-EE) oder aus Polyestern (TPC-EC) oder aus Polyethern (TPC-ET) bestehen. Zudem sind dort sogenannte Olefinelastomere (TPO) sowie Elastomere mit Ethylen/Propylen/Dien + Polypropylen (TPO-EPDM+PP) und solche mit Ethylen/Vinylacetat + Polyvinylidenchlorid (TPO-EVAC+PVDC) möglich.

Thermoplastische Styrolelastomere können als Styrol/Butadien-Block-Copolymer (TPS-SBS), als Styrol/Isopren-Block-Copolymer (TPS-SIS), als Styrol/Ethenbuten/Styrol-Block-Copolymer (TPS-SEBS) oder als Styrol/Ethenpropen/Styrol-Block-Copolymer (TPS-SEPS) ausgeführt sein.

Urethanelastomere (TPU) können enthalten: aromatische Hartsegmente und Polyester-Weichsegmente (TPU-ARES) oder aromatische Hartsegmente und Polyether-Weichsegmente (TPU-ARET) oder aromatische Hartsegmente und Weichsegmente mit Ether- und Esterbindungen (TPU-AREE) oder aromatische Hartsegmente und Polycarbonat-Weichsegmente (TPU-ARCE) oder aromatische Hartsegmente und Polycaprolacton-Weichsegmente (TPU-ARCL) oder aliphatische Hartsegmente und Polyester-Weichsegmente (TPU-ALES) oder aliphatische Hartsegmente und Polyether-Weichsegmente (TPU-ALET).

Beispielsweise können konkret die Stoffe "Carbothane®" (TPU) der Firma Lubrizol, "Barex®" der Firma IMEOS Barex, "ISOPLAST™" von Lubrizol Advanced Materials Inc. oder "Biresin®" der Firma Sika eingesetzt werden.

Thermoplastische Elastomere mit vernetztem Kautschuk (TPV) können hochvernetztes EPDM+BP enthalten (TPV-(EPDM-X+PP)) oder als hochvernetzter Acrylnitril/Butadien-Kautschuk + PP (TPV-(NBR-X+PP)) oder als hochvernetzter Naturkautschuk + PP (TPV-(NR-X+PP)) oder als hochvernetzter epoxidierter Naturkautschuk + PP (TPV-(ENR-X+PP)) oder als vernetztes Polybutylacrylat + PP (TPV-(PBA-X+PP)) ausgeführt sein.

Die thermoplastischen Elastomere weisen im Wesentlichen die gummielastischen Eigenschaften vernetzter Elastomere auf und verbinden diese mit dem Vorteil der thermoplastischen Verarbeitbarkeit.

Grundsätzlich können diese aus einer thermoplastischen Polymermatrix mit vernetzten oder unvernetzten Elastomerpartikeln als Weichphase bestehen (sogenannte Polymerblends). Sie können jedoch auch als sogenannte Pfropf- oder Copolymere ausgeführt sein, die in einem Polymermolekül thermoplastische Sequenzen und elastomere Sequenzen enthalten, die sich lokal entmischen können, so dass die thermoplastischen Sequenzen physikalische Vernetzungspunkte in der kontinuierlichen Matrix der elastomeren Sequenzen bilden (Beispiel: Styrol-Block-Copolymer (TPS)).

Bei der Gebrauchstemperatur befinden sich die elastomeren Sequenzen oberhalb ihrer Glastemperatur, während die thermoplastischen Sequenzen sich unterhalb ihrer Glastemperatur (bei amorphen Polymeren) bzw. der Schmelztemperatur (bei teilkristallinen Polymeren) befinden.

## Copolyamide (TPA)

Zur Herstellung von Copolyamiden können Lactam, Dihydroxipolytetrahydrofuran und Dicarbonsäure (TPA-ET) verwendet werden, zur Herstellung von TPA-EE: Laurinlactam, Adipinsäure und Bishydromethylcyclohexan.

## Thermoplastische Elastomere mit Copolyestern (TPC)

Zur Herstellung von TPC (thermoplastischen Elastomeren mit Copolyestern) können Weichsegmente aus Polyalkylenetherdiolen und/oder langkettigen aliphatischen Dicarbonsäureestern mit teilkristallinen PBT-Segmenten eingesetzt werden. Die entsprechenden Stoffe sind hydrolysefest und temperaturbeständig.

## Polyolefinelastomere (TPO)

Polyolefinelastomere (TPO) können auf der Basis von isotaktischen PP- und Ethylenpropylen-Kautschuken (EPDM) als Blends hergestellt werden.

Das thermoplastische Olefinelastomer TPO-(EVAC-PVDC) wird auf der Basis von Legierungen mit PVDC-Hartdomänen und einer teilvernetzten weichen EVAC-Colpolymer-Matrix hergestellt.

## Thermoplastische Elastomere mit Polystyrol (TPS)

Diese können beispielsweise als Dreiblock-Copolymere TPS-SBS ausgeführt werden, indem durch anionische Polymerisation, z. B. mit Lithiumbutyl, nacheinander Blöcke aus Styrol und Butadien und wahlweise noch einmal aus Styrol aufgebaut werden können. Ähnlich lassen sich Polymere vom Typ SIS (I = Isopren) herstellen (TPS-SIS). Die Polystyrol-Kettenabschnitte aggregieren dabei zu den harten Domänen und die Polybutadien-Kettenabschnitte zu flexiblen Kautschukbereichen.

TPS können auch im Mehrkomponenten-Spritzguss eingesetzt werden. Eine gute Haftung wird beispielsweise mit folgenden Kunststoffen erreicht: PE, PP, PS, ABS, PA, PPO, PBT (Abkürzungen vgl. Saechtling, Kunststofftaschenbuch, 29. Auflage 2004, Verlag Hansa, München/Wien).

Solche Stoffe können im Spritzguss dann mit dem ersten Werkstoff beispielsweise als Verstärkungsstrukturen verbunden werden.

## Polyurethanelastomere (TPU)

TPU können als Block-Copolymere durch Polyaddition langkettiger Diole (Kettenverlängerer: 1,4-Butandiol oder 1,6-Hexandiol, langkettige Diole: Polyetherdiole, Polyesterdiole, Polycarbonatdiole) mit aliphatischen Diisocyanaten hergestellt werden. Es sind Stoffe, die sich durch hohe Verschleißfestigkeit und Flexibilität sowie chemische Beständigkeit und hohe bioverträglichkeit auszeichnen.

TPV, Polyolefinblends mit vernetztem Kautschuk

Diese Stoffe weisen vernetzte Kautschuk-Weichsegmente auf und sind dadurch sehr elastisch.

Weitere thermoplastische Elastomere

Als weitere thermoplastische Elastomere kommen solche auf der Basis von PVC mit NBR oder PBA in Frage (NBR[TPZ-(NBR+PVC)] bzw. PBA [TPZ-(PBA+PVC)]).

Eine weitere vorteilhafte Ausführungsform der Erfindung sieht vor, dass der erste Werkstoff als Natur- oder Synthesekautschuk, insbesondere als R-Kautschuk, als M-Kautschuk, als O-Kautschuk, als Q-Kautschuk, als T-Kautschuk oder als U-Kautschuk ausgebildet ist.

Entsprechende Kautschuke können vorteilhaft auch mit Weichmachern eingesetzt und mit Öl gestreckt werden.

Zu den einzelnen Kautschukvarianten:

R-Kautschuke

Natur-Kautschuk (NR) weist eine hohe Festigkeit und Elastizität auf. Er ist temperaturstabil und elastisch bei geringer mechanischer Dämpfung.

Es kann vorteilhaft auch Isopren-Kautschuk (IR) mit oft noch höherer Elastizität eingesetzt werden.

Auch der Einsatz von Butadien-Kautschuk (BR) ist möglich. Es kann hier beispielsweise auch ein carboxylgruppenhaltiger Butadien-Kautschuk eingesetzt werden.

Vorteilhaft kann auch ein Chloropren-Kautschuk (CR) eingesetzt werden, insbesondere auch ein Styrolchloropren-Kautschuk.

Auch der Einsatz von Styrolbutadien-Kautschuk (SBR) kann vorteilhaft sein. Es kann hier beispielsweise ein carboxylgruppenhaltiger Styrolbutadien-Kautschuk eingesetzt werden.

Auch der Einsatz von Nitrilbutadien-Kautschuk (NBR), Nitrilchloropren-Kautschuk (NCR) und Butylkautschuk (IIR, CIIR, BIIR) mit IIR als Copolymerisat ist denkbar. Zudem kann auch ein Isoprenstyrol-Kautschuk (SIR) oder ein Polynorbonen-Kautschuk (PNR) eingesetzt werden. Auch der Einsatz von Transpolyoktenamer-Kautschuk (TOR) oder hydriertem NBR-Kautschuk (HNBR) ist denkbar.

M-Kautschuke

Unter den M-Kautschuken können beispielsweise Ethylenpropylen(dien)-Kautschuke (EPM, EPDM) eingesetzt werden. EPM kann dabei mit Peroxiden vernetzt, EPDM auch mit Schwefel vulkanisiert werden.

Es können auch Ethylen-Acrylester-Kautschuke (AECM), Ethylen-Vinylacetat-Kautschuke (EAM), chlorsulfonierter PE-Kautschuk (CSM), chlorierter PE-Kautschuk (CM), Acrylat-Kautschuk (ACM (AEM, ANM)), FluorKautschuk (FKM), Propylentetrafluorethylen-Kautschuk (FPM) oder Perfluor-Kautschuk (FFKM) eingesetzt werden.

O-Kautschuke

Als O-Kautschuke sind Bichlorhydrinhomopolymer-, Copolymer- und Terpolymer-Kautschuke (CO, ECO, ETER) denkbar. Epichlorhydrinelastomere können mit Aminen vernetzt sein. Vinylgruppenhaltige Terpolymere sind auch mit Schwefel oder Peroxid vulkanisierbar.

Auch der Einsatz eines Propylenoxid-Kautschuks (PO) ist denkbar.

Q-Kautschuke

Als Silikon-Kautschuke (Q-Kautschuke) sind Polydimethylsiloxan-Kautschuk (MQ), Methylphenylsiloxan-Kautschuk (MPQ), Methylvinylsiloxan-Kautschuk (VMQ), Methylphenylvinylsiloxan-Kautschuk (PVMQ), Methylfluorsiloxan-Kautschuk (MFQ) sowie Fluorsilikon-Kautschuk (MVFQ) und Flüssigsilikon-Kautschuk (LSR) einsetzbar. Silikon-Kautschuke weisen Ketten mit Siloxangruppen auf und sind physiologisch unbedenklich. Sie weisen eine hohe Dauerelastizität auf.

T-Kautschuke

Als T-Kautschuke mit Schwefel sind PolysulfidKautschuke (TM, ET) einsetzbar sowie Thiocarbonyldifluorid-Copolymer-Kautschuk (TCF).

U-Kautschuke

Als U-Kautschuke sind Nitrose-Kautschuk (AFMU), Urethan-Kautschuk sowie Polyester/Polyether-Kautschuk (EU, AU) einsetzbar. Die Verarbeitung von Urethan-Kautschuk kann bei thermoplastischem PUR-Elastomeren durch thermoplastische Umformung oder allgemein durch Gießen eines reaktionsfähigen Gemisches oder die Herstellung eines Prepolymeren und Einmischen von Füllstoffen und Vernetzern mit anschließender Vulkanisation in beheizten Formen hergestellt werden. AU und EU weisen eine außerordentlich hohe Festigkeit, Flexibilität und Elastizität auf. Sie verbinden diese Eigenschaft mit einer hohen Verschleißfestigkeit. AU weist dabei eine bessere Wasserbeständigkeit auf als EU.

Zudem können Polyphosphazene (PNF, FZ, PZ) mit alternierenden Ketten mit Phosphor und Stickstoff, beispielsweise Fluorphosphazen-Kautschuk, eingesetzt werden. Zudem ist der Einsatz von Phosphazen-Kautschuk mit Fluoralkyl- oder Fluoroxylalkylgruppen sowie Phosphazen-Kautschuk mit Phenoxygruppen denkbar. Auch der Einsatz von Polyperfluortrimethyltriazin-Kautschuk (PFMT) ist denkbar.

Der erste Werkstoff kann vorteilhaft wenigstens einen Zuschlagstoff enthalten, der ihn mechanisch verstärkt.

Der Zuschlagstoff kann auch derart ausgestaltet bzw. in den Werkstoff eingebracht sein, dass er diesen mechanisch anisotrop macht. Dies kann beispielsweise durch Einlegen von Fasern, Gittern oder Netzen erreicht werden oder dadurch, dass eine anisotrope Wechselwirkung des Zuschlagstoffs mit den übrigen Bestandteilen des ersten Werkstoffs stattfindet. Der erste Werkstoff kann auch durch das Herstellungsverfahren des Rotors mit anisotropen mechanischen Eigenschaften versehen werden, beispielsweise durch anisotrope Vernetzungsvorgänge oder durch Einsatz von anisotropen Guss-, Spritzguss- oder Extrusions- bzw. Ziehverfahren.

Entsprechende Verstärkungsfasern können beispielsweise als Glasfasern, Kohlenstofffasern, Kunststofffasern oder Naturfasern ausgebildet sein. Diese können beispielsweise gemeinsam in einer Vorzugsrichtung orientiert oder zumindest parallel zueinander angeordnet sein.

Glasfasern können beispielsweise in einer Länge von wenigen Mikrometern bis Millimetern eingesetzt werden.

Als Fasern können auch organische Werkstoffe wie Holzmehl, Zellstoff, Baumwollfasern, Baumwollgewebeschnitzel oder Seidenstränge eingesetzt werden. Zudem sind Kunstseidenstränge, mineralische Fasern, Kurz-und Langglasfasern sowie Glasmatten in der Verarbeitung durch Pressen, Spritzpressen und Spritzgießen mit verarbeitbar.

Als Kohlenstofffasern können karbonisierte und graphitierte CF-Verstärkungsfasern aus der Polyacrylnitritfaser verwendet werden. Es können auch Kohlenstoffspinnfasern verwendet werden, die als Monofilamente und uni- oder multiaxiale textile Gebilde verfügbar sind. Die glatten Faseroberflächen können dabei durch eine oxidierende Behandlung aufgeraut werden.

Als Hochtemperaturkunststofffasern kommen PPTA, PAI oder PBI in Frage. Diese werden aus spezifischen Lösungsmitteln wie beispielsweise NMP = N-Methylpyrolidon gesponnen. PPTA-Verstärkungsfasern werden aus flüssigkristallinen Lösungen hochkristallin gesponnen.

Die beschriebenen Fasern, Gewebe und Zuschlagstoffe können mit den verschiedenen oben erwähnten Volumenstoffen wie Polyurethanen, thermoplastischen Elastomeren und Kautschuken vorteilhaft verbunden werden.

Die oben genannten Materialien eignen sich aufgrund ihrer Eigenstabilität insbesondere dafür, dass der Rotor den zweiten Zustand ohne Einwirkung äußerer Kräfte erreicht.

Dies ist besonders vorteilhaft in Fällen, in denen der Rotor im komprimierten ersten Zustand in ein menschliches Gefäß einführbar sein soll und anschließend, vor Beginn der Rotation, eigenständig eine bestimmte expandierte Lage annehmen soll, aus der heraus dann die Drehung des Rotors beginnt.

Hierbei ist vorzugsweise vorgesehen, dass der anfangs im zweiten Zustand rotierend angetriebene Rotor unter Fluidlast einen dritten Zustand einnimmt. Hierbei ist vorteilhaft, dass je nach Abstimmung des Materials der dritte Zustand genau definierbar ist, praktisch wie bei einem "Baseballhandschuh", der in eine Richtung komprimierbar ist und in der anderen Richtung zur Sicherstellung der bestmöglichen Funktion eine definierte "Endstellung" aufweist. Dies hat vorliegend den großen Vorteil, dass auch drehzahlunabhängig eine bestimmte Arbeitsstellung (dritter Zustand) des Rotors garantiert werden kann, was zur strömungstechnischen Anpassung/Auslegung wichtig sein kann. Dies ist vorzugsweise derart ausgelegt, dass die radiale Auslenkung (gemessen von dem Mittelpunkt der Achse des Rotors zu dem radial äußersten Punkt) eines Rotorblattes im Drehzahlbereich von 5000 U/min bis 40000 U/min im Wesentlichen gleich bleibt, vorzugsweise, dass $\varepsilon 2{\to}3 < 0{,}05$ besonders vorzugsweise $\varepsilon 2{\to}3 < 0{,}03$ beträgt, wobei $\varepsilon_{2\to3}$

definiert ist als $\varepsilon_{2\to3} = \dfrac{|r_2 - r_3|}{r_2}$ , wobei

$r_2$ = maximale radiale Ausdehnung im <u>zweiten</u> Zustand, gemessen vom Rotormittelpunkt zum radial äußersten Punkt;

$r_3$ = maximale radiale Ausdehnung im <u>dritten</u> Zustand, gemessen vom Rotormittelpunkt zum radial äußersten Punkt.

Eine weitere vorteilhafte Weiterbildung sieht vor, dass der Rotor so gestaltet ist, dass er bei Stillstand aus dem dritten Zustand elastisch reversibel in den zweiten Zustand zurückkehrt. Das heißt, dass diese Bewegung faktisch der "Hookeschen Geraden" gehorcht. Die Abstimmung des Materials ist hierbei einfacher als bei Gegenständen nach dem Stand der Technik, es handelt sich vorzugsweise um einen homogenen Kunststoffrotor bzw. einen Kunststoffrotor, bei dem metallische Einbettungen (oder Einbettungen aus anderem Material) gegeben sind. Die primäre Verformbarkeit erfolgt hierbei nicht durch Schwächungen im Fußbereich der Rotorblätter (also nabennah), sondern im Wesentlichen über den gesamten Rotor (die radiale Länge betrachtet). Dies macht es wiederum gut möglich, den Nabenbereich mit metallischen Einbettungen/ Streben zu verstärken, auch um eine definierte Strömungsform im Betrieb, selbst bei hohen Drehzahlen beispielsweise im Bereich von 5000 U/min bis 40000 U/min, zu gewährleisten.

Eine weitere vorteilhafte Weiterbildung sieht vor, dass der Rotor so gestaltet ist, dass die Einnahme des ersten Zustandes aus dem zweiten Zustand und die Einnahme des dritten Zustandes aus dem zweiten Zustand in entgegengesetzter Richtung erfolgen. Das heißt (siehe auch Diagramme, auf die weiter unten Bezug genommen wird), dass der erste Zustand im ersten Quadranten eines Spannungs-Dehnungs-Diagrammes erfolgt (Spannung = Ordinate, Dehnung = Abszisse) und der dritte Zustand im dritten Quadranten gegeben ist.

Diese Ausführungsform hat beträchtliche Vorteile, da hierdurch einerseits ein "Endanschlag" (sei es durch Materialeigenschaften oder mechanische Blockierung infolge eingebetteter Elemente) für den dritten Zustand erfolgen kann, um über ein Drehzahlband hinweg den gleichen Arbeitspunkt bereitzustellen. Andererseits hat dies den Vorteil, dass bei Inbetriebnahme des Rotors, insbesondere bei intraventrikulären Anwendungen, bei der der Zugriff durch medizinisches Personal direkt, beispielsweise in den Ventrikel eines Herzens, begrenzt ist, eine "automatische" Aufrichtung durch den Fluiddruck erfolgt. Dies ist ein entscheidender Vorteil gegenüber Vorrichtungen, bei denen der erste Zustand und der dritte Zustand beide im ersten Quadranten stattfinden. Bei solchen Vorrichtungen wäre nämlich die Aufstellkraft (für die Aufstellung vom ersten in den zweiten Zustand, also den unbewegten Zustand) derart stark zu wählen, dass selbst gegen einen aufgebrachten Fluiddruck eine sichere Aufstellung möglich ist. Dies kann, insbesondere in zeitkritischen Situationen bei der Wiederbelebung eines Patienten, ein entscheidender Nachteil sein.

Daher wird erfindungsgemäß in einer bevorzugten Ausführungsform gefordert, dass das selbstbestimmte Aufrichten des Rotors von einem komprimierten ersten Zustand in den expandierten zweiten Zustand beim Herausziehen aus der Schleuse innerhalb von maximal zehn Sekunden möglich ist und eine weitere Verformung in den dritten Zustand dann sofort erfolgen kann. Dies ist kräftemäßig auch günstiger als bei der oben genannten Alternative, bei der erster Zustand und dritter Zustand beide im ersten Quadranten sind, da Kriechvorgänge und Hysteresen bei der erfindungsgemäßen Variante deutlich besser sind.

Eine weitere vorteilhafte Weiterbildung sieht vor, dass der Rotor so gestaltet ist, dass er, ausgehend vom anfänglichen zweiten Zustand bei Überführung in den ersten Zustand, anschließend in den dritten Zustand und schließlich in den zweiten Zustand zurück, eine bleibende Restdehnung $\varepsilon_H$ von vorzugsweise weniger als 8 %, besonders vorzugsweise weniger als 3 %, ganz besonders vorzugsweise weniger als 1 % aufweist. $\varepsilon_H$ ist

hier definiert als $\quad \varepsilon_H = \dfrac{|r_4 - r_0|}{r_0}$ , , wobei

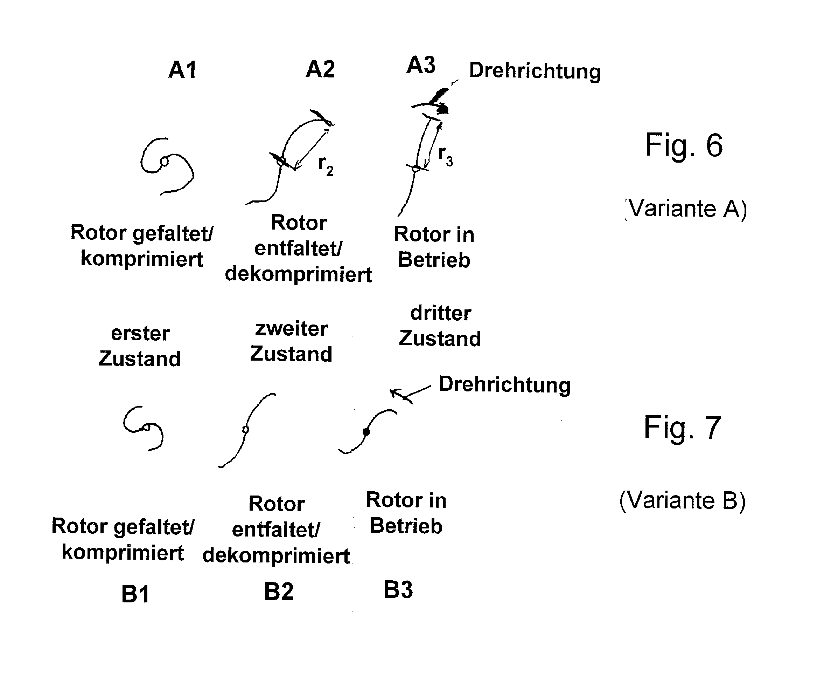

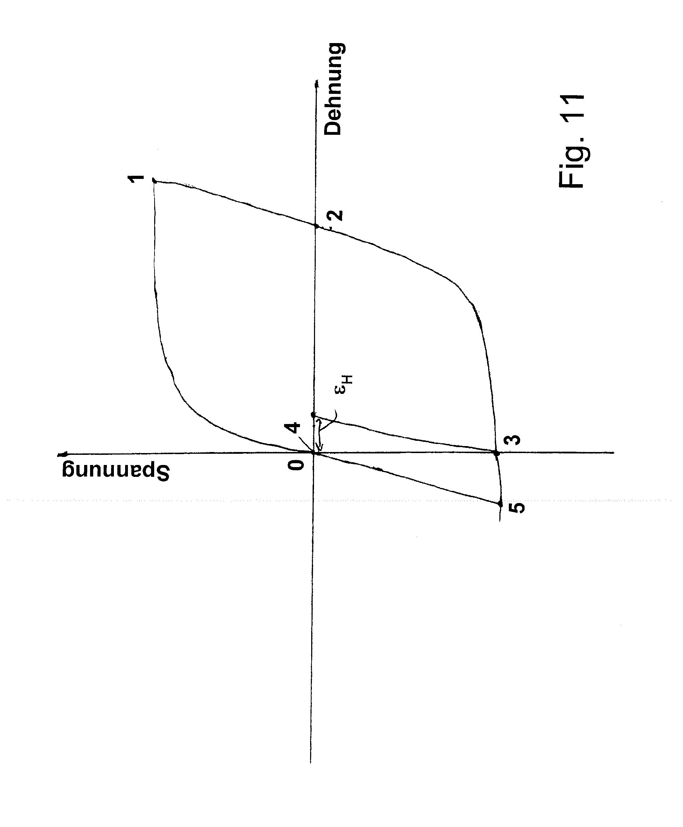

$r_4$ = maximale radiale Ausdehnung im zweiten Zustand (zum Endzeitpunkt, also am Ende, siehe Fig. 11), gemessen vom Rotormittelpunkt zum radial äußersten Punkt (Maßweise siehe Fig. 6);

$r_0$ = maximale radiale Ausdehnung im zweiten Zustand (zum Anfangszeitpunkt, siehe Fig. 11), gemessen vom Rotormittelpunkt zum radial äußersten Punkt (Maßweise s. Fig. 6).

Hierbei wird davon ausgegangen, dass der erste Zustand für 0 Stunden bis 12 Stunden besteht und der dritte Zustand für 0 Stunden bis 2000 Stunden besteht und die Temperatur stets zwischen 1°C und 50°C beträgt, vorzugsweise zwischen 18°C und 40°C.

Eine weitere vorteilhafte Weiterbildung sieht vor, dass der Rotor mindestens ein Rotorblatt aufweist, wobei das Rotorblatt eine Druckseite und eine Saugseite aufweist und die Druckseite einen konkaven Querschnitt hat oder maximal einen Wendepunkt aufweist. Die Druckseite ist hierbei die Seite, welche gegen den Fluiddruck, beispielsweise des Blutes im Ventrikel, arbeitet. Vorzugsweise handelt es sich bei der Geometrie des Rotorblattes um ein Rotorblatt, dessen Strömungsdruckseite in sämtlichen Querschnitten senkrecht bzw. parallel zur Rotationsachse konkav ist, gerade ist oder maximal einen Wendepunkt aufweist. Hierdurch ergibt sich eine hohe Effizienz des Rotors; eine geometrisch ungünstige und herstellungstechnisch problematische Aneinanderreihung von konvexen und konkaven Querschnitten ist somit nicht notwendig.

Die vorliegende Erfindung betrifft außerdem eine intraventrikuläre Blutpumpe enthaltend einen Rotor nach einem der vorhergehenden Beispiele sowie eine Schleuse zum Komprimieren des Rotors. Das Komprimieren des Rotors erfolgt hierbei durch Eindringen des Rotors in die Schleuse, vorzugsweise durch Eindringen des Pumpenkopfes mit dem darin befindlichen Rotor in die Schleuse, besonders vorzugsweise durch Einziehen des Pumpenkopfes mit dem Rotor in die Schleuse. Sowohl Schleuse als auch Rotor können hierbei aus den oben genannten Kunststoffen bestehen.

Um Adhäsionsvorgänge, Kriechvorgänge sowie Hysteresen zu vermeiden, hat es sich als vorteilhaft herausgestellt, dass Rotor und Schleuse zunächst ungefügt bereitgestellt werden. Das heißt, dass der Rotor, beispielsweise bei der Anlieferung vom Hersteller in ein Krankenhaus, nicht in der Schleuse komprimiert ist. Dies ermöglicht es, dass unmittelbar vor der Implantation der Rotor zunächst, beispielsweise in einer Versuchsapparatur, getestet wird und das medizinische Personal (dies ist auch aus Haftungsgründen unter Umständen wichtig) den Rotor somit prüft und dann selbst in die Schleuse einbringt bzw. einzieht. Nach Entfalten des Rotors im menschlichen Körper, beispielsweise in einem Ventrikel, wird dieser dann in Betrieb gesetzt. Hierbei ist vorteilhaft, dass es nicht zu einem Setzen und/oder Verkleben kommt; eine Funktionsprüfung ist möglich, und die Hysteresen werden vermindert. Vorzugsweise ist die Handhabung des Gerätes so, dass bei der Implantation des Rotors der Rotor maximal zehn Stunden in dem komprimierten ersten Zustand gelagert ist, vorzugsweise weniger als drei Stunden, ganz besonders vorzugsweise weniger als 30 Minuten.

Im Folgenden wird die Erfindung anhand eines Ausführungsbeispiels in einer Zeichnung gezeigt und anschließend beschrieben.

Dabei zeigt

Fig. 1 schematisch die Anordnung einer Herzkatheterpumpe in einer Herzkammer,

Fig. 2 eine Herzkatheterpumpe in vergrößerter Darstellung,

Fig. 3 einen Rotor einer Pumpe in dreidimensionaler Ansicht mit einer Nabe,

Fig. 4 einen nabenlosen Rotor in einer dreidimensionalen Ansicht,

Fig. 5 in einer dreidimensionalen Ansicht einen Rotor mit einer Mehrzahl von Schaufelblättern,

Fig. 6 eine schematische Veranschaulichung von Rotorverformungszuständen nach Variante A,

Fig. 7 eine schematische Veranschaulichung von Rotorverformungszuständen nach Variante B,

Fig. 8 ein Spannungs-Dehnungs-Diagramm für die in Fign. 6 und 7 gezeigten Zustände,

Fig. 9a den Zustand A1 aus Fig. 6,

Fign. 9b-9h Schnitte an unterschiedlichen axialen Stellen entlang der Rotorachse entsprechend Fig. 9a,

Fig. 10 eine Ansicht des in den Fign. 9a-9h gezeigten Rotors im Zustand A2 sowie

Fign. 11/12 Hysteresekurven.

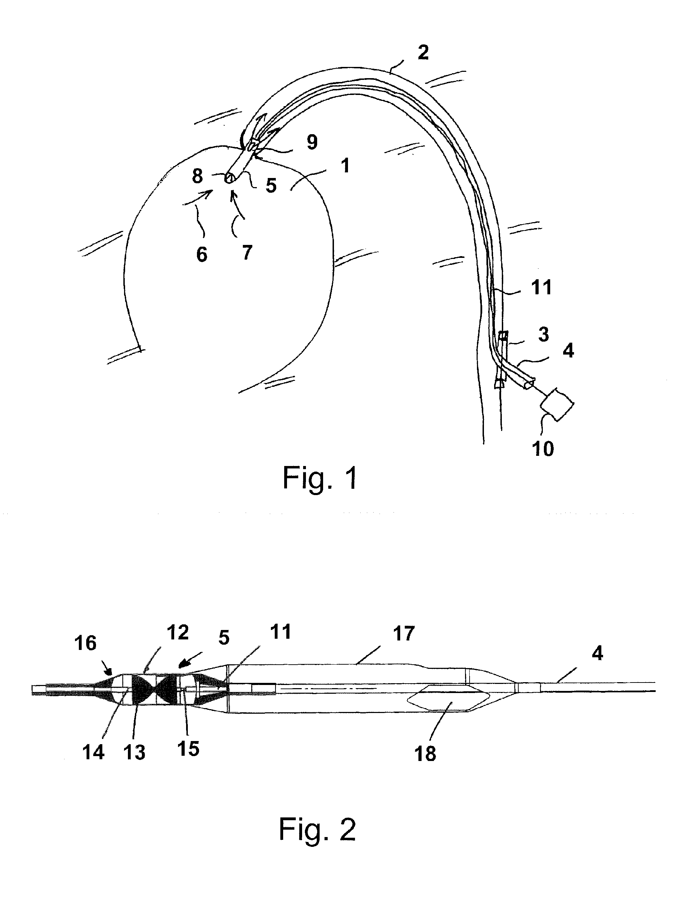

Fig. 1 zeigt in vereinfachter schematischer Darstellung ein Ventrikel 1 einer Herzkammer, in das ein Blutgefäß 2 (Aorta) mündet. Im Bereich der Herzklappe ist eine Pumpe 5 von dem Blutgefäß 2 aus in den Ventrikel 1 wenigstens teilweise eingeschoben. Die Pumpe 5 ist durch einen Hohlkatheter 4 mittels einer Schleuse 3 in das Körperinnere eines Patienten und durch das Blutgefäß 2 bis in die Herzkammer vorgeschoben. Innerhalb des Hohlkatheters 4 verläuft eine Antriebswelle 11, die mittels eines außerhalb des Körpers vorgesehenen Motors 10 antreibbar ist und die selbst den Rotor der Pumpe 5 antreibt.

Die Pumpe 5 weist einen Ansaugkäfig 8 an ihrer vorderen, distalen Seite auf, durch den, symbolisiert durch die Pfeile 6, 7, Blut angesaugt wird. Dies wird durch Öffnungen 9 am proximalen Ende der Pumpe bzw. eines Abströmschlauches in das Blutgefäß 2 ausgestoßen. Durch die Förderfunktion der Pumpe 5 unterstützt diese die Herzpumptätigkeit oder ersetzt sie teilweise.

Damit die Pumpe 5 innerhalb des Ventrikels 1 nach dem Transport durch das Blutgefäß 2 radial expandierbar ist, sind in dem dargestellten Beispiel sowohl der Rotor als auch das Pumpengehäuse bzw. der Abströmschlauch radial komprimierbar und expandierbar.

Diese sind in der Fig. 2 näher dargestellt. Innerhalb eines Pumpengehäuses 12, das ein aufweitbares Maschengeflecht aufweisen und mit einer dichten Membran bespannt sein kann, ist ein Rotor 13 angeordnet, der ein schraubenförmiges Schaufelblatt und eine Nabe aufweist. Die Nabe kann in Lagern an ihrem proximalen Ende 15 sowie dem distalen Ende 14 gelagert sein.

Am distalen Ende des Gehäuses 12 ist ein Ansaugkäfig 16 angeordnet. Durch diesen wird Blut angesaugt. Am distalen Ende der Pumpe 5 ist ein sogenanntes Pigtail 19 angeordnet, das als flexible Fortsetzung einen Abstandhalter bildet, um im Saugbetrieb oder beim Transport das Anstoßen an Herzwände oder Gefäßwände sowie das Ansaugen an Innenflächen zu verhindern und die Lage der Pumpe zu stabilisieren.

Die Nabe 14, 15 ist am proximalen Ende der Pumpe mit der flexiblen antreibbaren Welle 11 verbunden.

Über das Gehäuse 12 der Pumpe ist ein Abströmschlauch 17 gezogen, in den die Pumpe 5 das Blut pumpt und durch den es an der Herzklappe vorbei in die Aorta bzw. das Blutgefäß 2 fließen kann. Durch Ausströmöffnungen 18 wird es dort aus dem Abströmschlauch in das Blutgefäß ausgestoßen.

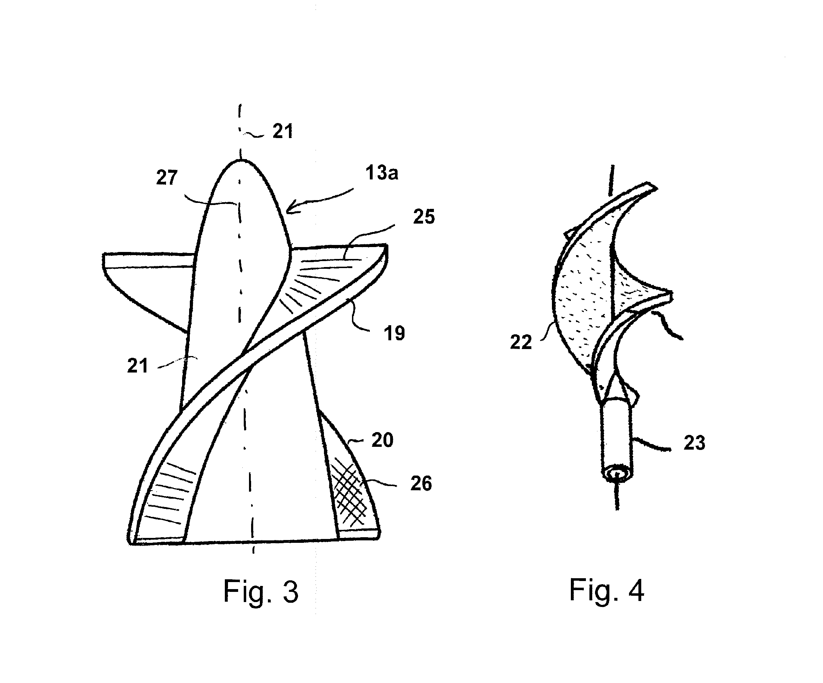

Der Rotor 13 ist in der Fig. 3 näher dargestellt. Er weist eine Nabe 13a aus einem thermoplastischen Elastomer auf, mit der zwei schraubenartig ineinander gewundene Schaufelblätter 19, 20 einstückig verbunden sind. Diese sind im expandierten Zustand dargestellt, den sie im Betrieb unter der Kraftwirkung des Fluidgegendrucks einnehmen. Die Schaufelblätter sind im komprimierten ersten Zustand an die Nabe 13a fast vollständig anklappbar. Die Materialelastizität der Schaufeln sowie der Nabe reicht hierfür aus, und das Material ist derart beschaffen, dass die entsprechende Verformung reversibel ist. Der Verformungshub und die kraftfreie, entspannte Stellung sind vorteilhaft derart bemessen, dass sich das Material möglichst über den gesamten Hub entlang einer hysteresefreien Spannungskurve dehnen lässt.

Der Rotor ist somit derart ausgelegt, dass die auftretenden Schub-, Zug- bzw. Druckverformungen innerhalb des proportionalen Bereichs der hookschen Geraden stattfinden. Dies ist durch geeignetes Design und einen entsprechend gewählten Betriebspunkt der Verformung zu erreichen.

In der Fig. 3 sind Verstärkungsfasern 25 angedeutet, die in etwa radial, von der Rotationsachse der Nabe 13a aus gesehen, verlaufen und die Schaufelblätter 19, 20 verstärken.

Zusätzlich zu den radial verlaufenden Verstärkungsfasern können auch rechtwinklig hierzu verlaufende Verstärkungsfasern vorgesehen sein, die mit den ersten Verstärkungsfasern auch zu einem flächigen Gewebe verwoben sein können.

Es sind an dem Schaufelblatt 20 beispielhaft auch Verstärkungsfasern 26 dargestellt, die als Gewebe aus zwei Gruppen von senkrecht zueinander verlaufenden Fasern ausgebildet sind, wobei alle Fasern in einem Winkel von beispielsweise 45° zur Rotationsachse 27 verlaufen.

Die Verstärkungsfasern 25, 26 können beispielsweise als Glasfasern oder als Polyamidfasern bzw. Kohlenstofffasern ausgebildet sein. Sie werden bei der Herstellung des Rotors entweder dem Spritzgusswerkstoff bei der Extrusion beigemischt, insbesondere dann, wenn es sich um kurze Fasern handelt, die nicht notwendigerweise orientiert sein müssen, oder sie werden in eine Spritzguss- oder Gussform eingelegt und mittels eines Werkstoffs umspritzt. Dieser Werkstoff kann beispielsweise ein Polyurethan, ein thermoplastisches Elastomer oder ein Kautschuk sein.

In der Fig. 4 ist ein nabenloser Rotor dargestellt, bei dem ein einziges Schaufelblatt 22 mit einem Wellenende 23 verbunden und durch dieses antreibbar ist. Das Schaufelblatt 22 kann beispielsweise durch Nanopartikel verstärkt sein, die in den ersten Werkstoff eingebettet sind und dann einen Bestandteil des ersten Werkstoffes bilden. Auch ein nabenloser Rotor kann, wie anhand der Fig. 3 dargestellt, mit entsprechenden Fasern verstärkt sein.

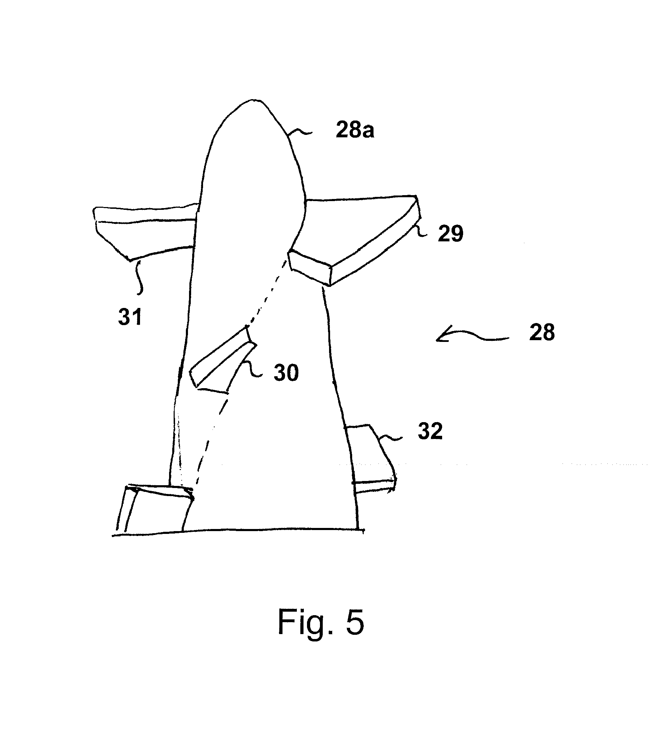

Die Fig. 5 zeigt einen Rotor 28 mit Förderschaufeln 29, 30, 31, 32, die alle einzeln an der Nabe 28a angeordnet und befestigt sind. Eine derartige separate Anordnung von Förderschaufeln an der Nabe bewirkt eine einfachere Anklappbarkeit an die Nabe und damit eine einfachere Kompression des Rotors.

Die einzelnen Schaufelblätter 29, 30, 31, 32 können jeweils aus demselben elastomeren Material bestehen und auch einstückig mit der Nabe zusammenhängen. Sie können mittels eines pulver-, granulat- oder faserförmigen Zuschlagstoffs bis zu der gewünschten Steifheit verstärkt sein.

Auch das Material der Nabe 28a kann durch Einfügen von Verstärkungsfasern oder anderen Zuschlagstoffen verstärkt sein.

Fig. 6 zeigt schematisch eine bevorzugte Ausführungsform der Erfindung in Hinblick auf die Verformungszustände des Rotors. Gezeigt sind hierbei jeweils einblättrige Rotoren (d. h. Rotoren, bei denen ein Rotorblatt zu beiden Seiten der Achse absteht). In den Fign. 6 und 7 ist hierbei eine Draufsicht auf die Achse (die Achse ragt aus der Papierebene hinaus) gezeigt; die Achse ist hierbei mit einem kleinen Kreis gekennzeichnet.

In Fig. 6 (im Folgenden auch Variante A genannt) ist die gefaltete/komprimierte Ausgangsstellung des Rotors (auch A1 genannt) gezeigt. Dies ist beispielsweise die Ausgangsstellung des Rotors (erster Zustand), bei der er in eine Schleuse eingezogen ist.

Der zweite Zustand (Zustand A2) zeigt den vom Fluiddruck noch unbelasteten, entfalteten/dekomprimierten Rotor. Dieser Zustand tritt beispielsweise auf, wenn eine intraventrikuläre Blutpumpe nach dem Einführen in das menschliche Herz aus der Schleuse entfernt wurde.

Der dritte Zustand (A3) ist der Zustand, bei dem sich der Rotor bewegt. Hierbei erfolgt eine Drehung des Rotors in Fig. 6 im Uhrzeigersinn. Es ist deutlich, dass hierdurch eine noch stärkere Verformung in den "ausgeklappten" Zustand erfolgt, es erfolgt quasi eine "Selbststabilisierung" des Rotors. Somit ist der Arbeitspunkt genau einstellbar, beispielsweise durch einen Anschlag und/oder durch entsprechende Auslegung des Materials.

Der Ausgangszustand ist wiederum B1, nach der Entfaltung ergibt sich der Zustand B2. Das Fördern von Fluid erfolgt allerdings hier entgegen dem Uhrzeigersinn, so dass der Rotor eher radial wieder eingeklappt wird. Dies bedeutet, dass die Entfaltungskraft zwischen den Zuständen B1 und B2 so groß sein muss, dass auch der Fluidförderbetrieb den Rotor nicht derart einfallen lässt, dass dieser nicht mehr ordentlich fördern kann.

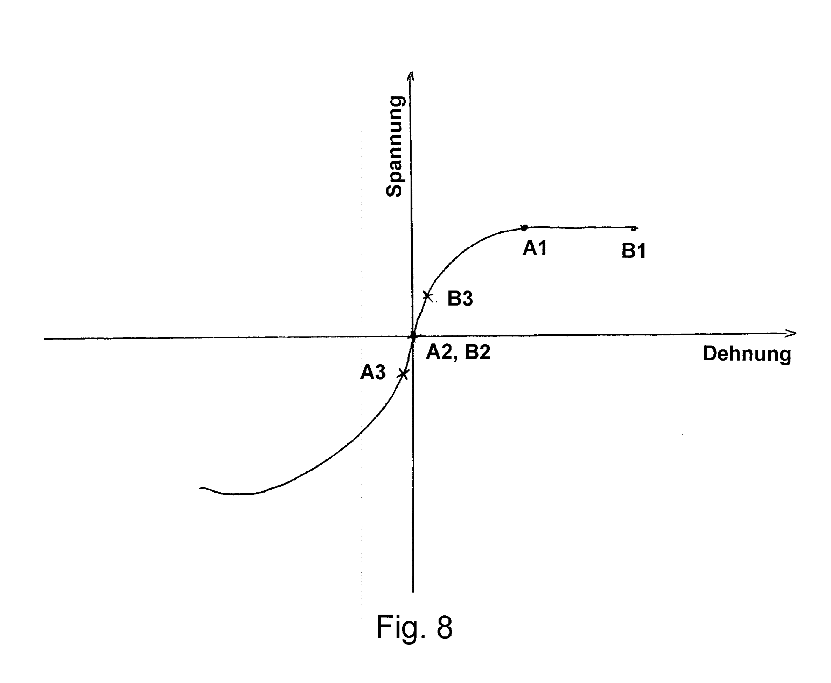

Diese geometrischen Verhältnisse werden in Fig. 8 nochmals verdeutlicht. Im gezeigten Diagramm ist die Dehnung auf der Abszisse und die Spannung auf der Ordinate gezeigt. A1 bzw. B1 ist hier im ersten Quadranten gezeigt. Beim Entnehmen des Rotors aus der Schleuse ergeben sich die kraftlosen Zustände A2 bzw. B2. Im Förderbetrieb ergibt sich dann eine Verformung zu A3 bzw. B3. Hierbei ist festzuhalten, dass A3 im dritten Quadranten, B3 dagegen wiederum im ersten Quadranten ist. Das heißt, dass bei der bevorzugten Ausführungsform der Erfindung A1 und A3 jeweils in diagonalen Quadranten stehen, während B1 und B3 (in der weniger bevorzugten Variante) im selben Quadranten angeordnet sind.

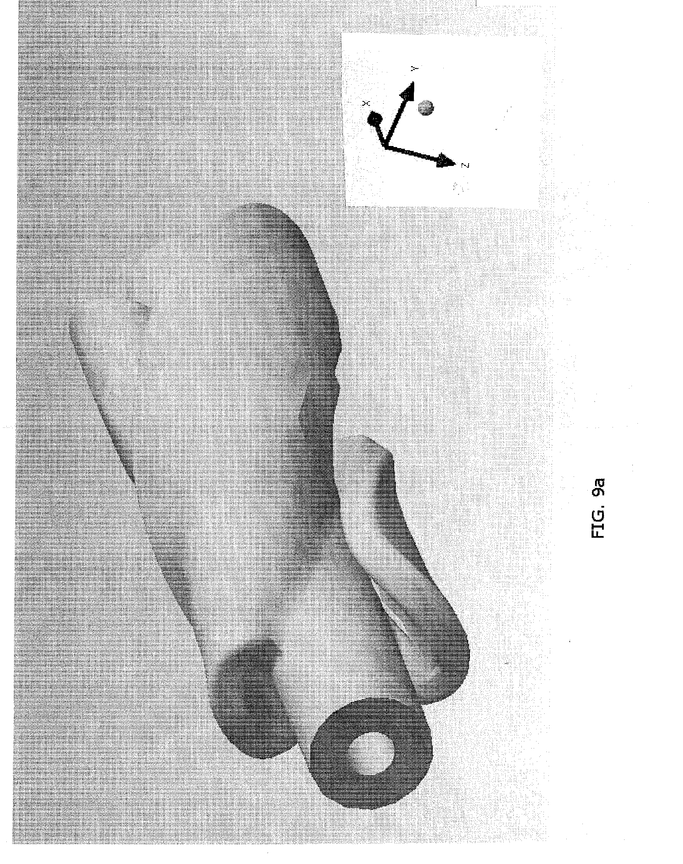

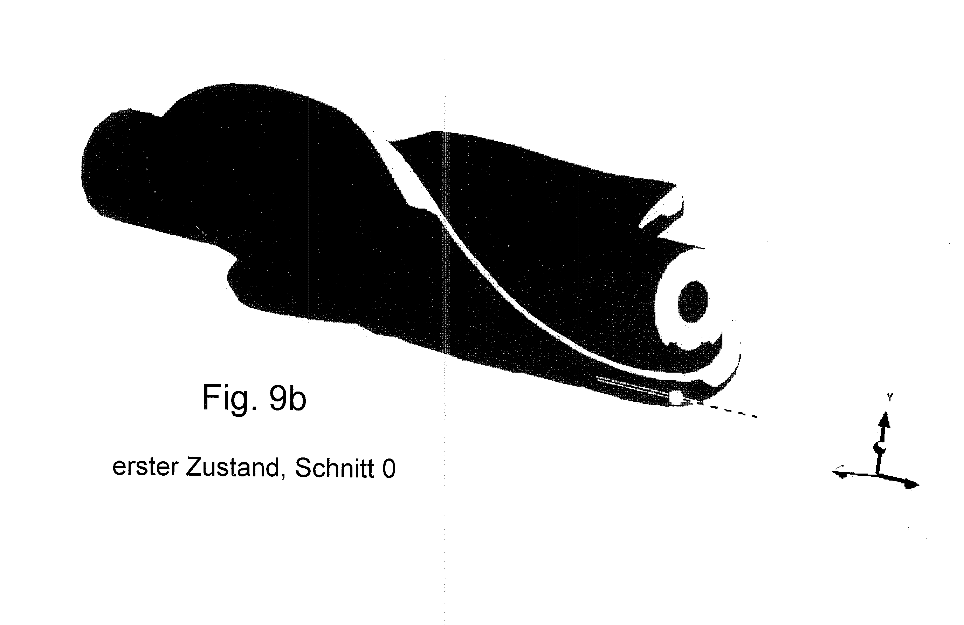

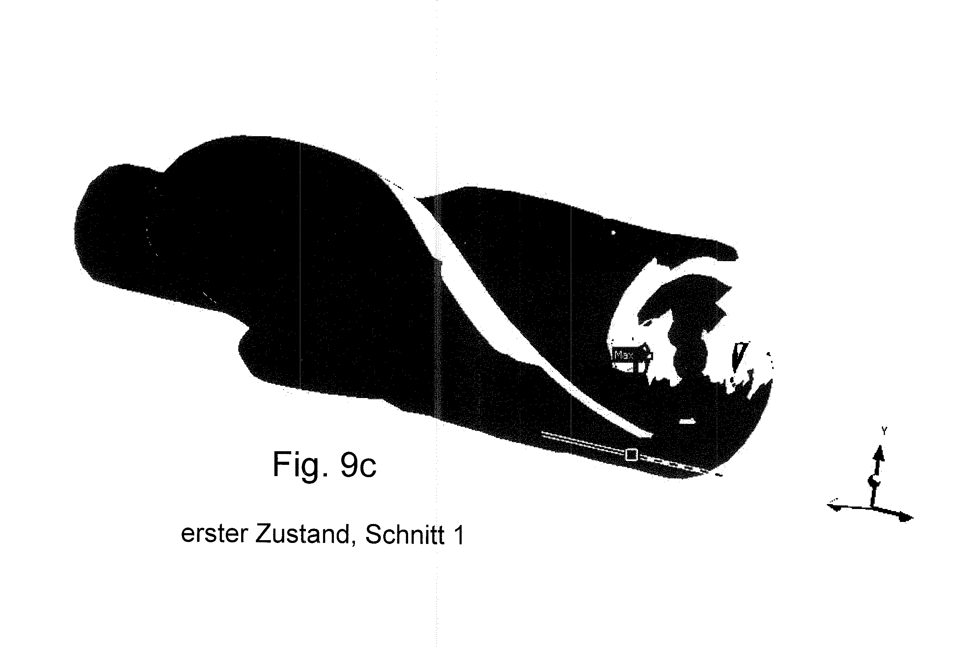

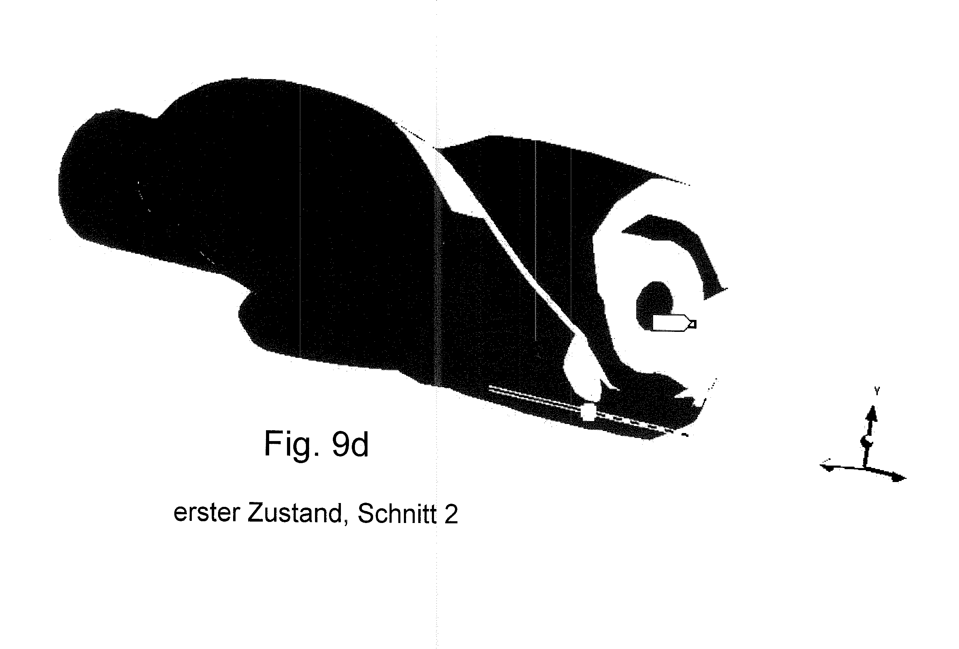

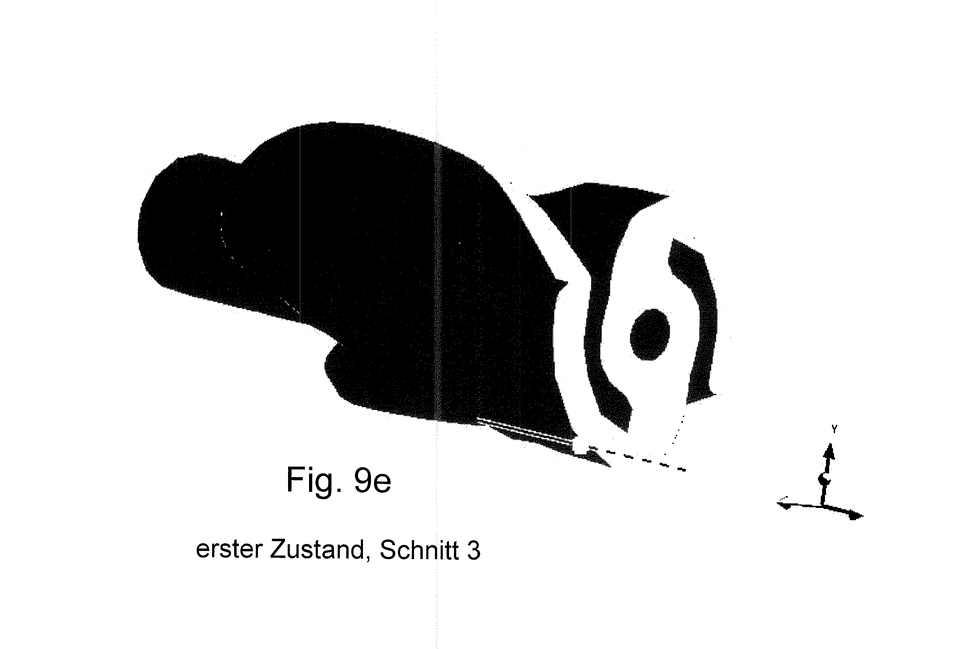

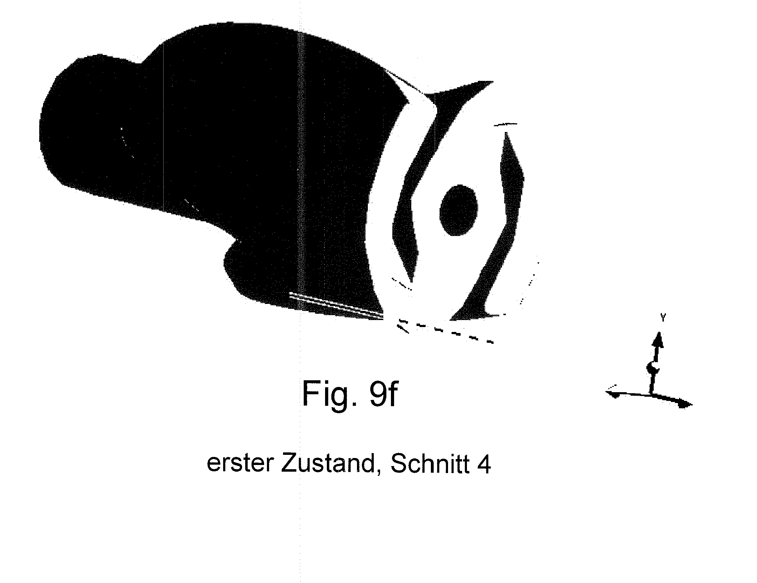

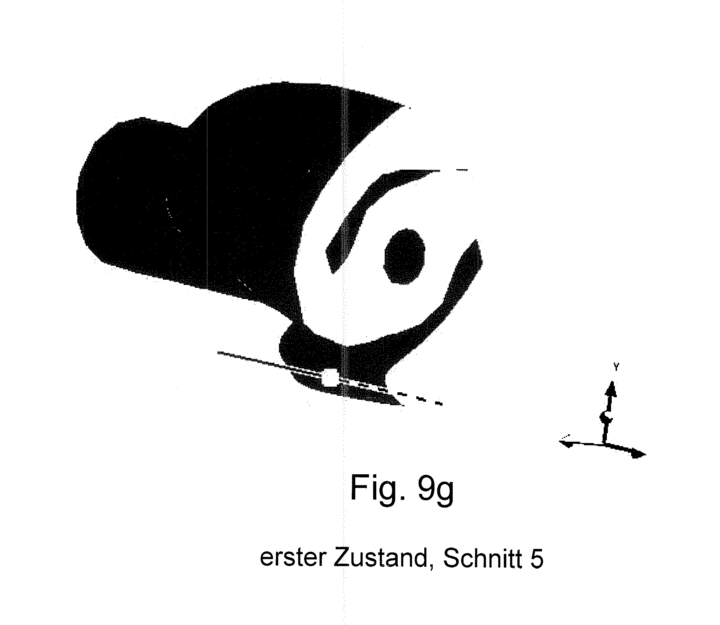

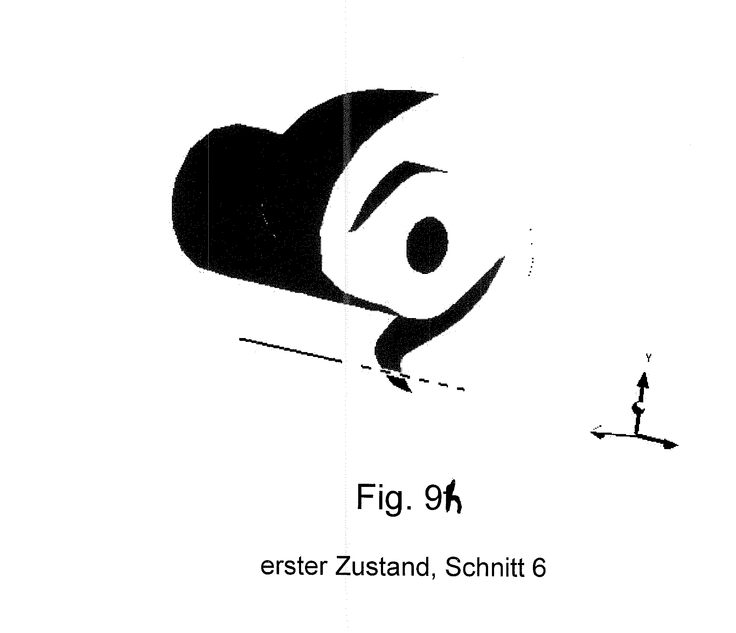

Eine komprimierte Ausführungsform (erster Zustand, "A1", siehe auch Fig. 6) wird in Fig. 9a gezeigt. Hierbei ist die x-Achse in Richtung der Rotorachse gezeigt. Anhand der folgenden Figuren wird beispielhaft die elastische Vergleichsdehnung (nach von Mises) offenbart. Als Rotor ist wiederum der oben genannte einblättrige Rotor (das Rotorblatt ist durch die Nabe getrennt) gezeigt. Fign. 9b-9h zeigen hierbei unterschiedliche Schnittebenen, wobei zum Ausdruck kommt, dass durch die hier gewählte Geometrie die maximalen Vergleichsdehnungen gering bleiben, was zu einer hysterese- und kriecharmen Verwendung führt.

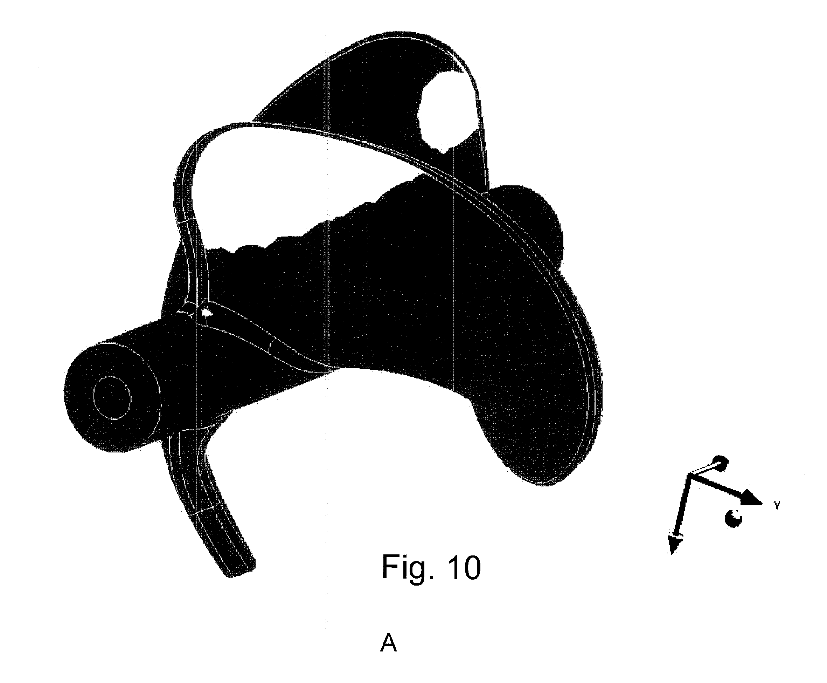

Zum Vergleich ist in Fig. 10 noch einmal der zweite Zustand "A2" gezeigt.

Fig. 11 zeigt einen Teil einer typischen Hysteresekurve eines Werkstoffs, der nach einer entsprechenden Verformung wieder entlastet wird. Der mit "0" bezeichnete Punkt ist der Ausgangszustand des unbelasteten Werkstücks. Punkt "1" entspricht dem als komprimierten Rotor bezeichneten Punkt. Wird der Rotor nun in diesem Punkt entlastet, das heißt die Spannung auf Null reduziert, so bleibt eine bleibende Verformung (Punkt "2") bestehen, die hier mehr als 50% der maximalen Dehnung des Werkstoffes im komprimierten Zustand ausmacht. Der Rotor würde also nicht mehr die Ursprungsform einnehmen. Erst durch eine weitere Belastung entgegen der ursprünglichen Belastungsrichtung würde der Rotor wieder seine ursprüngliche Form einnehmen (Punkt "3"). Diese Belastung müsste Materialspannungen erzeugen, die in ihrer Größe annähernd der ursprünglichen Belastung entsprechen. Die Erzeugung entgegengesetzter Spannungen dieser Größenordnung allein durch den Fluiddruck ist jedoch für eine Blutpumpe kaum realistisch, da dann erhebliche schädigende Kräfte auf das Blut einwirken würden. Bei Entlastung in diesem Zustand würde der Rotor eine bleibende Verformung (Punkt "4") behalten. Damit wäre ein Zustand gegeben, der nicht wiederholbare Verhältnisse für die Handhabung einer solchen Blutpumpe schafft. Erst durch eine weitere Erhöhung dieser (negativen) Spannungen ist es möglich, den mit "5" bezeichneten Punkt der Kurve zu erreichen, von dem aus bei Entlastung wieder der Ausgangszustand "0" erreichbar ist, bei dem der Rotor im entlasteten Zustand seine Ausgangsform einnimmt.

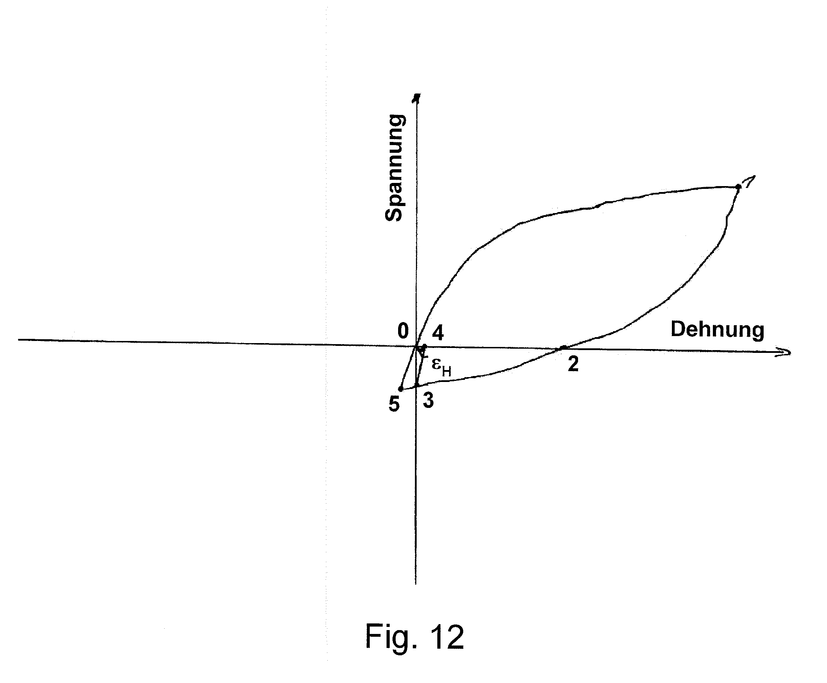

Fig. 12 zeigt das Verformungsverhalten eines Werkstoffs, der eine relativ geringe Hysterese zeigt. Die Punkte "0" bis "5" entsprechend der Abbildung in Fig. 11. Aufgrund der geringeren bleibenden Verformungen wäre hier eher ein beherrschbares Verhalten des Rotors realisierbar, da die geringeren bleibenden Verformungen weniger wesentliche bzw. unwesentliche Auswirkungen auf das Verhalten in der Praxis haben. Ideal für die Anwendung ist jedoch ein Werkstoff, der überhaupt keine Hysterese aufweist und der Kurve "0"-"1" auch bei Entlastung folgen würde. Am ehesten ist ein solches Verhalten erreichbar bzw. annähernd erreichbar, wenn man bei der Auslegung im Bereich der Hookeschen Geraden bleibt. Für die zuverlässige Funktion eines solchen Rotors ist es also wesentlich entscheidender, dass der Werkstoff im Bereich der auftretenden Verformungen ein hysteresearmes, idealerweise hysteresefreies Verhalten zeigt, als dass der Rotor eine Änderung der Kennliniensteigerung aufweist. Insbesondere ist es entscheidend, dass die verbleibende Dehnung nach Wegfall Komprimierung(Punkt 2) in praktisch relevanter Zeit weniger als 50% bevorzugt weniger als 25% der maximalen Dehnung des Werkstoffes im ersten Zustand ausmacht.

Aspekte der Erfindung sind unter anderem:

1. Rotor (13) für eine Pumpe, mit wenigstens einem Schaufelblatt (19, 20, 22, 29, 30, 31, 32), wobei der Rotor zur Förderung eines Fluids in axialer oder radialer Richtung um eine Rotationsachse (21) rotierend antreibbar ist und wobei der Rotor in radialer Richtung zwischen einem ersten, radial komprimierten Zustand und einem zweiten, radial expandierten Zustand reversibel elastisch verformbar ist.

2. Rotor nach Aspekt 1, dadurch gekennzeichnet, dass der Rotor wenigstens teilweise aus einem ersten, elastischen Werkstoff in Form eines Schaumstoff-Polyurethans, eines massiven Polyurethans, eines thermoplastischen Elastomers, eines Kautschuks oder eines superelastischen Werkstoffs, insbesondere superelastischen Polymers, besteht.

3. Rotor nach Aspekt 2, dadurch gekennzeichnet, dass der erste Werkstoff ein Polyurethan auf der Basis eines Diisocyanats umfasst.

4. Rotor nach Aspekt 3, dadurch gekennzeichnet, dass der erste Werkstoff mit einem Polyetherpolyol hergestellt ist.

5. Rotor nach Aspekt 3 oder 4, dadurch gekennzeichnet, dass der erste Werkstoff mit einem organisch gefüllten Polyol, insbesondere einem Pfropf-, SAN- oder Polymerpolyol oder einem PHD-Polyol, hergestellt ist.

6. Rotor nach Aspekt 2, dadurch gekennzeichnet, dass der erste Werkstoff als thermoplastisches Elastomer, insbesondere als Polyamid-TPE, als Copolyester-TPE, als Styrol-TPE, als Urethan-TPE, oder als thermoplastisches Elastomer mit vernetztem Kautschuk ausgebildet ist oder einen solchen Stoff umfasst.

7. Rotor nach Aspekt 2, dadurch gekennzeichnet, dass der erste Werkstoff als Natur- oder Synthesekautschuk, insbesondere als R-Kautschuk, als M-Kautschuk, als O-Kautschuk,
als Q-Kautschuk, als T-Kautschuk oder als U-Kautschuk ausgebildet ist oder einen solchen Stoff umfasst

8. Rotor nach Aspekt 2 oder einem der folgenden, dadurch gekennzeichnet, dass der erste Werkstoff wenigstens einen Zuschlagstoff enthält, der den ersten Werkstoff mechanisch verstärkt.

9. Rotor nach Aspekt 2 oder einem der folgenden, dadurch gekennzeichnet, dass der erste Werkstoff einen Zuschlagstoff enthält, der den Werkstoff mechanisch anisotrop macht.

10. Rotor nach Aspekt 2 oder einem der folgenden, dadurch gekennzeichnet, dass der erste Werkstoff durch das Herstellungsverfahren des Rotors anisotrope mechanische Eigenschaften aufweist.

11. Rotor nach Aspekt 2 oder einem der folgenden, dadurch gekennzeichnet, dass der erste Werkstoff Verstärkungsfasern, insbesondere Glasfasern, Kohlenstofffasern, Kunststofffasern oder Naturfasern aufweist.

12. Rotor nach Aspekt 11, dadurch gekennzeichnet, dass die Fasern nach einer Vorzugsrichtung orientiert sind.

13. Rotor nach Aspekt 2 oder einem der folgenden, dadurch gekennzeichnet, dass der erste Werkstoff mit Nanopartikeln gefüllt ist.

14. Rotor nach Aspekt 1, dadurch gekennzeichnet, daß der Rotor den zweiten Zustand ohne Einwirkung äußerer Kräfte einnimmt.

15. Rotor nach Aspekt 14, dadurch gekennzeichnet, dass der anfangs im zweiten Zustand rotierend angetriebene Rotor unter Fluidlast einen dritten Zustand einnimmt.

16. Rotor nach Aspekt 15, dadurch gekennzeichnet, dass der Rotor so gestaltet ist, dass er bei Stillstand aus dem dritten Zustand elastisch reversibel in den zweiten Zustand zurückkehrt.

17. Rotor nach Aspekt 14 oder 15, dadurch gekennzeichnet, dass dieser so gestaltet ist, dass die Einnahme des ersten Zustands aus dem zweiten Zustand und die Einnahme des dritten Zustands aus dem zweiten Zustand in entgegengesetzter Richtung erfolgen.

18. Rotor nach Aspekt 15, dadurch gekennzeichnet, dass der Rotor, ausgehend vom anfänglichen zweiten Zustand bei Überführung in den ersten Zustand, anschließend in den dritten Zustand und schließlich in den zweiten Zustand zurück, eine bleibende Restdehnung ($\varepsilon_H$) von vorzugsweise weniger als 8 %, besonders vorzugsweise weniger als 3 %, ganz besonders vorzugsweise weniger als 1 % aufweist. Hierbei wird davon ausgegangen, dass der erste Zustand für 0 Stunden bis 12 Stunden bestand und der dritte Zustand für 0 Stunden bis 2000 Stunden bestand und die Temperatur stets zwischen 1˚C und 50˚C, vorzugsweise zwischen 18˚C und 40˚C betrug.

19. Rotor nach einem der vorhergehenden Aspekte, dadurch gekennzeichnet, dass der Rotor mindestens ein Rotorblatt hat, wobei das Rotorblatt eine Druckseite und eine Saugseite aufweist und die Druckseite einen monoton konvexen Querschnitt aufweist.

20. Intraventrikuläre Blutpumpe enthaltend einen Rotor nach einem der vorhergehenden Aspekte sowie eine Schleuse, wobei diese Schleuse derart gestaltet ist, dass beim Eindringen des Rotors in die Schleuse der Rotor zumindest radial komprimiert wird.

21. Verfahren zum Bereitstellen einer intraventrikulären Blutpumpe nach Aspekt 21, dadurch gekennzeichnet, dass Schleuse und Rotor zunächst ungefügt bereitgestellt werden und der Rotor erst unmittelbar vor der Implantation in einen menschlichen oder tierischen Körper in die Schleuse eingebracht wird. Vorteil: Kein Setzen des Materials, kein Verkleben des Rotors mit der Hülle/Schleuse, eine Funktionsprüfung vor Implantation ist möglich, auch durch die kurze Zeit werden Fließ- bzw. Hystereseeffekte minimiert.

**Patentansprüche**

1.  Rotor (13) für eine Pumpe, mit wenigstens einem Schaufelblatt (19, 20, 22, 29, 30, 31, 32), wobei der Rotor zur Förderung eines Fluids in axialer oder radialer Richtung um eine Rotationsachse (21) rotierend antreibbar ist und wobei der Rotor in radialer Richtung zwischen einem ersten, radial komprimierten Zustand und einem zweiten, radial expandierten Zustand reversibel elastisch verformbar ist.

2.  Rotor nach Anspruch 1, **dadurch gekennzeichnet, dass** der Rotor wenigstens teilweise aus einem ersten, elastischen Werkstoff in Form eines Schaumstoff-Polyurethans, eines massiven Polyurethans, eines thermoplastischen Elastomers, eines Kautschuks oder eines superelastischen Werkstoffs, insbesondere superelastischen Polymers, besteht.

3.  Rotor nach Anspruch 2, **dadurch gekennzeichnet, dass** der erste Werkstoff ein Polyurethan auf der Basis eines Diisocyanats umfasst.

4.  Rotor nach Anspruch 3, **dadurch gekennzeichnet, dass** der erste Werkstoff mit einem Polyetherpolyol hergestellt ist.

5.  Rotor nach Anspruch 3 oder 4, **dadurch gekennzeichnet, dass** der erste Werkstoff mit einem organisch gefüllten Polyol, insbesondere einem Pfropf-, SAN- oder Polymerpolyol oder einem PHD-Polyol, hergestellt ist.

6.  Rotor nach Anspruch 2, **dadurch gekennzeichnet, dass** der erste Werkstoff als thermoplastisches Elastomer, insbesondere als Polyamid-TPE, als Copolyester-TPE, als Styrol-TPE, als Urethan-TPE, oder als thermoplastisches Elastomer mit vernetztem Kautschuk ausgebildet ist oder einen solchen Stoff umfasst und/oder
dass der erste Werkstoff als Natur- oder Synthesekautschuk, insbesondere als R-Kautschuk, als M-Kautschuk, als O-Kautschuk, als Q-Kautschuk, als T-Kautschuk oder als U-Kautschuk ausgebildet ist oder einen solchen Stoff umfasst.

**7.** Rotor nach Anspruch 2 oder einem der folgenden, **dadurch gekennzeichnet, dass** der erste Werkstoff wenigstens einen Zuschlagstoff enthält, der den ersten Werkstoff mechanisch verstärkt und/oder dass der erste Werkstoff einen Zuschlagstoff enthält, der den Werkstoff mechanisch anisotrop macht und/oder

dass der erste Werkstoff durch das Herstellungsverfahren des Rotors anisotrope mechanische Eigenschaften aufweist und/oder

dass der erste Werkstoff Verstärkungsfasern, insbesondere Glasfasern, Kohlenstofffasern, Kunststofffasern oder Naturfasern aufweist, wobei vorzugsweise die Fasern nach einer Vorzugsrichtung orientiert sind, und/oder dass der erste Werkstoff mit Nanopartikeln gefüllt ist.

**8.** Rotor nach Anspruch 1, **dadurch gekennzeichnet, dass** der Rotor den zweiten Zustand ohne Einwirkung äußerer Kräfte einnimmt.

**9.** Rotor nach Anspruch 8, **dadurch gekennzeichnet, dass** der anfangs im zweiten Zustand rotierend angetriebene Rotor unter Fluidlast einen dritten Zustand einnimmt.

**10.** Rotor nach Anspruch 9, **dadurch gekennzeichnet, dass** der Rotor so gestaltet ist, dass er bei Stillstand aus dem dritten Zustand elastisch reversibel in den zweiten Zustand zurückkehrt.

**11.** Rotor nach Anspruch 8 oder 9, **dadurch gekennzeichnet, dass** dieser so gestaltet ist, dass die Einnahme des ersten Zustands aus dem zweiten Zustand und die Einnahme des dritten Zustands aus dem zweiten Zustand in entgegengesetzter Richtung erfolgen.

**12.** Rotor nach Anspruch 9, **dadurch gekennzeichnet, dass** der Rotor, ausgehend vom anfänglichen zweiten Zustand bei Überführung in den ersten Zustand, anschließend in den dritten Zustand und schließlich in den zweiten Zustand zurück, eine bleibende Restdehnung ($\varepsilon_H$) von vorzugsweise weniger als 8 %, besonders vorzugsweise weniger als 3 %, ganz besonders vorzugsweise weniger als 1% aufweist.

**13.** Rotor nach einem der vorhergehenden Ansprüche, **dadurch gekennzeichnet, dass** der Rotor mindestens ein Rotorblatt hat, wobei das Rotorblatt eine Druckseite und eine Saugseite aufweist und die Druckseite einen monoton konvexen Querschnitt aufweist.

**14.** Intraventrikuläre Blutpumpe enthaltend einen Rotor nach einem der vorhergehenden Ansprüche sowie eine Schleuse, wobei diese Schleuse derart gestaltet ist, dass beim Eindringen des Rotors in die Schleuse der Rotor zumindest radial komprimiert wird.

**15.** Verfahren zum Bereitstellen einer intraventrikulären Blutpumpe nach Anspruch 14, **dadurch gekennzeichnet, dass** Schleuse und Rotor zunächst ungefügt bereitgestellt werden und der Rotor erst unmittelbar vor der Implantation in einen menschlichen oder tierischen Körper in die Schleuse eingebracht wird.

Fig. 1

Fig. 2

Fig. 3

Fig. 4

EP 2 407 186 A1

**28a**

**29**

**31**

**28**

**30**

**32**

Fig. 5

**A1**　　　**A2**　　　**A3**　⌒ Drehrichtung

Fig. 6

(Variante A)

$r_2$

$r_3$

Rotor gefaltet/ komprimiert　　Rotor entfaltet/ dekomprimiert　　Rotor in Betrieb

erster Zustand　　zweiter Zustand　　dritter Zustand

⌒ Drehrichtung

Fig. 7

(Variante B)

Rotor gefaltet/ komprimiert　　Rotor entfaltet/ dekomprimiert　　Rotor in Betrieb

**B1**　　　**B2**　　　**B3**

EP 2 407 186 A1

Fig. 8

FIG. 9a

Fig. 9b

erster Zustand, Schnitt 0

Fig. 9c

erster Zustand, Schnitt 1

Fig. 9d

erster Zustand, Schnitt 2

Fig. 9e

erster Zustand, Schnitt 3

Fig. 9f

erster Zustand, Schnitt 4

Fig. 9g

erster Zustand, Schnitt 5

Fig. 9h

erster Zustand, Schnitt 6

Fig. 10

Fig. 11

Fig. 12

**EUROPÄISCHER RECHERCHENBERICHT**

Europäisches Patentamt
European Patent Office
Office européen des brevets

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X<br><br><br>Y | US 2006/062672 A1 (MCBRIDE MARK W [US] ET AL MCBRIDE MARK W [US] ET AL)<br>23. März 2006 (2006-03-23)<br>* Absatz [0004] - Absatz [0005] *<br>* Absatz [0029] - Absatz [0034] *<br>* Absatz [0041] *<br>* Absätze [0075], [0081] *<br>* Abbildungen 3A,3B *<br>----- | 1-3,<br>8-11,13,<br>14<br>4,6,7 | INV.<br>A61M1/10<br>A61M1/12 |
| X | US 2008/114339 A1 (MCBRIDE MARK W [US] ET AL) 15. Mai 2008 (2008-05-15)<br>* Absatz [0084] - Absatz [0092] *<br>----- | 1-3 | |
| Y | JP 7 242723 A (MITSUBISHI CABLE IND LTD)<br>19. September 1995 (1995-09-19)<br>* Absätze [0010], [0038] *<br>----- | 4,6 | |
| Y | WO 2010/063494 A1 (ECP ENTWICKLUNGSGMBH [DE]; SCHECKEL MARIO [DE])<br>10. Juni 2010 (2010-06-10)<br>* Seite 6, Absatz 1 *<br>----- | 7 | RECHERCHIERTE SACHGEBIETE (IPC)<br><br>A61M |

Der vorliegende Recherchenbericht wurde für alle Patentansprüche erstellt

| Recherchenort | Abschlußdatum der Recherche | Prüfer |
|---|---|---|
| München | 20. Dezember 2010 | Bichlmayer, K |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
.......................................................................................................
& : Mitglied der gleichen Patentfamilie, übereinstimmendes
    Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**  EP 10 07 5303

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

20-12-2010

| Im Recherchenbericht angeführtes Patentdokument | Datum der Veröffentlichung | Mitglied(er) der Patentfamilie | Datum der Veröffentlichung |
|---|---|---|---|
| US 2006062672 A1 | 23-03-2006 | AU 2005286914 A1<br>CN 101278127 A<br>EP 1789314 A2<br>US 2009060743 A1<br>WO 2006034158 A2 | 30-03-2006<br>01-10-2008<br>30-05-2007<br>05-03-2009<br>30-03-2006 |
| US 2008114339 A1 | 15-05-2008 | AU 2007230945 A1<br>CA 2646277 A1<br>EP 1996252 A2<br>JP 2009530041 T<br>WO 2007112033 A2 | 04-10-2007<br>04-10-2007<br>03-12-2008<br>27-08-2009<br>04-10-2007 |
| JP 7242723 A | 19-09-1995 | KEINE | |
| WO 2010063494 A1 | 10-06-2010 | EP 2194278 A1 | 09-06-2010 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20080114339 A1

**In der Beschreibung aufgeführte Nicht-Patentliteratur**

- **SAECHTLING.** Kunststofftaschenbuch. Verlag Hansa, 2004, vol. 29